# EUROPEAN PATENT APPLICATION

(11) **EP 2 919 175 A1**
(43) Date of publication of application: **16.09.2015**
(21) Application number: 15158402.6
(22) Date of filing: 10.03.2015
(51) Int. Cl.: G06Q 10/10, G06Q 50/24

(54) **METHOD AND SYSTEM FOR AUTOMATED MANAGEMENT OF PATIENTS' CLINICAL DATA AND ACCESS TO SAID CLINICAL DATA IN EMERGENCY CONDITIONS**

(30) Priority: 10.03.2014 IT MI20140372
(71) Applicant: Avionord S.r.l., 20138 Milano (IT)
(72) Inventor: CREMASCOLI, EUGENIO, I-20145 MILANO (IT)
(74) Representative: Baroni, Matteo

(57) **Abstract**

The present invention relates to a method for automated management of patients' clinical data and sanitary emergencies through a system of electronic processing units interconnected via the Internet network, said system comprising a server processing unit, the method being characterized in that it comprises the steps of:
- providing a user registration system assigning access credentials and a sanitary identification card; said access credentials include a first level of read-only credentials and a second level of read/write credentials,
- providing access to clinical information stored in said server processing unit (51) by means of personal processing units (52, 53, 54), via said Internet network and said first and second levels of credentials,
- storing said clinical information in a multilingual format, allowing said registered users to choose one of said languages for the read/write operations to be executed in said server processing unit (51),
- providing a system for emergency management and assisted return calling through said personal processing units (52, 53, 54) with said first or second level of access credentials.

## Description

### Field of the invention

The present invention relates to a method and a system for automated management of patients' clinical data and for allowing access to said clinical data in sanitary emergency conditions, including the possibility of activating a sanitary return.

In general, the present invention concerns the management of and access to medical data associated with a clinical or medical record of a patient, i.e. a user of the service, for allowing access to that patient's sanitary documents and information, in particular by using a connection network such as the Internet.

### Background art

In the current state of the art, there is still no effective flow of patients' information among the various segments of the sanitary sector. It is hard for physicians to rebuild and document a patient's medical record, especially when treatments were given in different hospital structures. The task of building and updating his/her medical history, therefore, falls upon the patient him/herself. However, very often the patient cannot comply with this duty, and physicians must visit patients whose medical history they do not adequately know.

This lack of information flow turns out to be particularly unfavourable in emergency situations, so that the patient is admitted to the emergency room without the doctor knowing the patients' clinical history or any previous medical treatments or pathologies in progress.

It is known to provide on-line access to clinical records, wherein the patient gains access to his/her clinical record and sanitary data, typically by secure login through a user identifier and a PIN or a password. Such on-line clinical records, however, have not been effectively implemented in widespread systems available to the users.

The solutions known in the art are not very effective in emergency situations, when it is not possible for the user to gain access to his/her clinical record in order to show it to the rescuers.

In such emergency cases, admission to the emergency room occurs with no knowledge of the patient's data and of his/her preliminary medical conditions. This may cause many problems, even resulting in a wrong diagnosis. As a matter of fact, administration of drugs contraindicated for the patient is a serious problem.

A further problem is the lack of an effective and fast emergency calling and intervention system, especially from remote locations.

### Summary of the invention

It is one object of the present invention to provide a method and a system for automated management of patient's clinical data and for managing such data in the event of sanitary emergencies, which overcomes the problems suffered by the prior art.

It is also one object of the present invention to allow access to a patient's clinical record from any access terminal, such as a laptop or desktop computer or a tablet, whereon the patient's clinical history can be retrieved by using connection means such as an Internet browser.

It is another object of the present invention to improve the patient's sanitary treatment, particularly in emergency conditions, by providing the doctor in the emergency room with the patient's medical history, including any previous diagnoses, prescriptions, vaccinations, allergies and surgical operations, even when the patient is unconscious or is in a foreign country and does not speak the local language.

It is a further object of the present invention to give the patient the possibility of keeping his/her medical data up-to-date. It will thus be possible, with accurate medical data, to make better diagnoses and treatments and to avoid replicating tests already made.

In general, the present invention relates to a method and a system that improve the protection of a patient's health.

These and other objects are achieved through a system for gaining access to a patient's data as set out in the appended claims, which are an integral part of the present description.

The present invention discloses a method for automated management of patients' clinical data, and for allowing access to said clinical data in the event of sanitary emergencies, through a system of electronic processing units interconnected via the Internet network, said system comprising a server processing unit, the method being characterized in that it comprises the steps of:
- providing a user registration system assigning access credentials and a sanitary identification card; said access credentials including a first level of read-only credentials and a second level of read/write credentials,
- providing access to clinical information stored in said server processing unit by means of personal processing units, via said Internet network and said first and second levels of credentials,
- storing said clinical information in a multilingual format, allowing said registered users to choose one of said languages for the read/write operations to be executed in said server processing unit,
- providing a system for emergency management and assisted return calling through said personal processing units, with said first or second level of access credentials.

Further particular and advantageous aspects will become more apparent from the following detailed description and from the dependent claims, which are an integral part of the present description.

### Brief description of the drawings

Some preferred and advantageous embodiments will now be described by way of non-limiting example with reference to the annexed drawings, wherein:
- Figure 1 exemplifies a first login screen of the system according to the present invention.
- Figure 2 exemplifies a first patient data display screen according to the present invention.
- Figure 3 exemplifies a second login screen of the system according to the present invention.
- Figure 4 exemplifies a second patient data display screen according to the present invention.
- Figure 5 is a block diagram of the system according to the invention.
- Figure 6 shows one example of embodiment of the sanitary card.
- Figure 7 shows an example of an operational flow chart of the system's software. The drawings show different aspects and embodiments of the present invention and, where appropriate, similar structures, components, materials and/or elements are designated in the various drawings by the same reference numerals.

### Detailed description of some embodiments of the invention

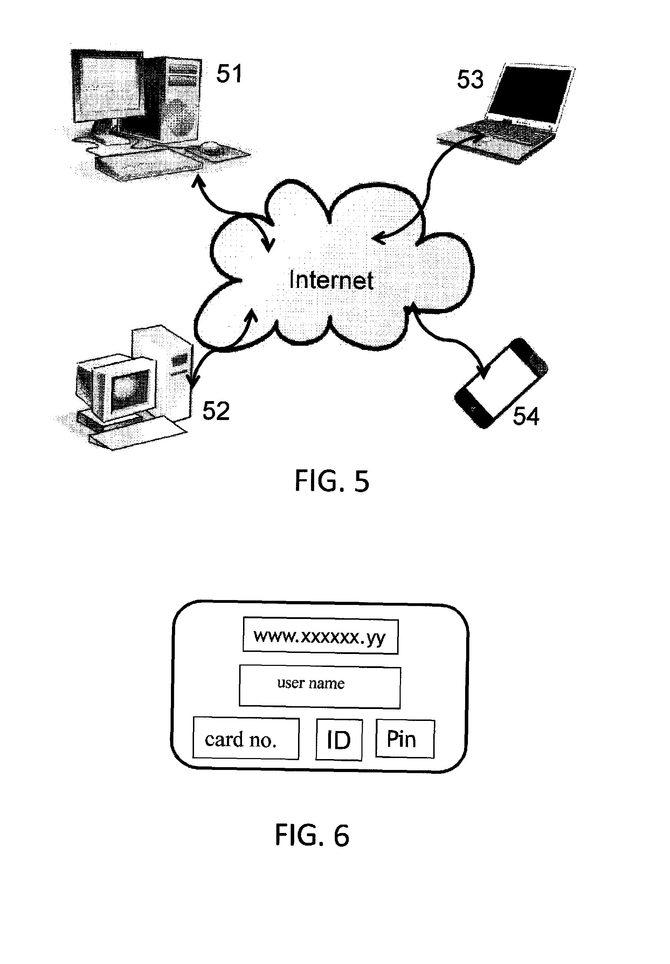

A non-limiting example of the electronic system of the invention (Figure 5) includes an electronic processing unit or center 51 acting as a server for the management of functions and clinical data, as well as for assisted sanitary return. The server has bidirectional access to a communication network, e.g. the Internet, through a web browser.

The hardware system includes means for intercommunication with remote personal electronic units, such as personal computers, whether desktop computers 52 or laptop computers 53, or personal digital assistants 54, so long as they are provided with software application management functions and with suitable hardware for entering, storing, transmitting, receiving and displaying data, especially clinical data.

The remote palmtop unit can handle emergency calls and is provided with a GPS function for identifying its position, and hence the position of the user.

The hardware system also includes sanitary cards in the form of plastic identification cards, to be assigned to each user registered in the system. It is preferable that every user always brings with him/her his/her own sanitary card, so that it can be used correctly and quickly, especially in the event of a sanitary emergency situation.

The sanitary card (Figure 6) contains identification data of the user and of the card itself (user name, card number), data allowing access to the management system (ID, PIN), and the Internet address (*www.xxxxxx.yy*) of the management server.

The method envisages the definition of the following types of bidirectional access to the system, protected by user identifiers and passwords:
- access as manager, essentially reserved to the server electronic processing unit or center;
- access as hospital unit or medical emergency unit, whether fixed (hospital) or mobile (ambulance), essentially by means of remote units, such as personal computers situated at said hospital unit or medical emergency unit;
- access as user, through remote units, such as personal computers, especially laptops and personal digital assistants, by users being in places allowing access to the Internet network, wherever in the world.

The method of the invention envisages an initial step in which the user registers into the system. During the registration step, the user is assigned an ID card like the one previously described.

The user then enters the system; he/she will be allowed to set a new password, but the initial one will not be deleted and will remain highlighted on the card, to be used for managing the various access credentials. It is recommended that the user always bring the card with him/her, so that it will be readily available in sanitary emergency situations.

At least the following levels of system access credentials are envisaged:
- a first level, with which access to the system can be gained for data read operations only, or for using the assisted return function, by means of the identifiers shown on the card and usable by anyone having the possibility of reading the card, i.e. not just the user, but also, for example, sanitary staff for emergency or routine assistance operations, also at fixed or mobile hospital units;
- a second level, with which access can be gained to the system for data read/update operations, or for using the assisted return function; this level is only available to the user upon entry of a modified PIN, as described above;
- a third level, for system management operations carried out by staff working at the server station.

Each user has the possibility of entering into the system data that characterize him/her from a clinical viewpoint. The data will be available in the server for display and/or editing.

After having registered, the user will enter the system by connecting to the server via the Internet network, update his/her own identifier (PIN), and can then enter the following types of data, which may still be updated afterwards:
- personal identification;
- family doctor;
- people to call in case of emergency;
- personal and familiar clinical anamnesis: for example, such data may include: smoker, alcohol consumption, diabetes, hypertension, handicaps, organ donor, etc.;
- clinical data: for example, blood group, RH, transfusions, pacemaker, etc.;
- allergies;
- regularly used drugs, and posology thereof;
- surgical operations already undergone;
- clinical history, including all pathologies;
- vaccinations;
- and more...

The user can also enter and display scanned or original clinical reports and documents.

The data can be entered, and then viewed, in entry screens containing subsequent rows with fixed definitions of the data that need to be entered, with adjacent fields allowing the user to freely enter further qualifying data.

The system can provide a multilingual service: field definitions and some general information are translated into various languages, at least including the most internationally widespread ones (Italian, English, French, German, Spanish, Russian, Chinese, etc.).

This makes what is shown on the screen more easily and quickly understood, while also reducing the probability of evaluation mistakes in emergency conditions, especially by medical staff who, if the user is abroad, may not be familiar with the system language normally used by the user.

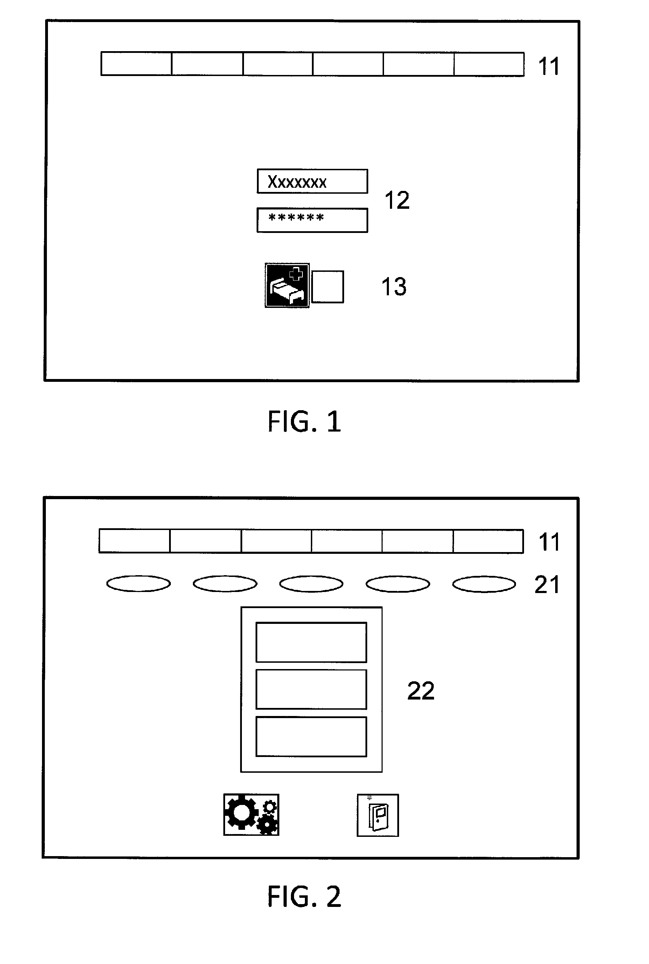

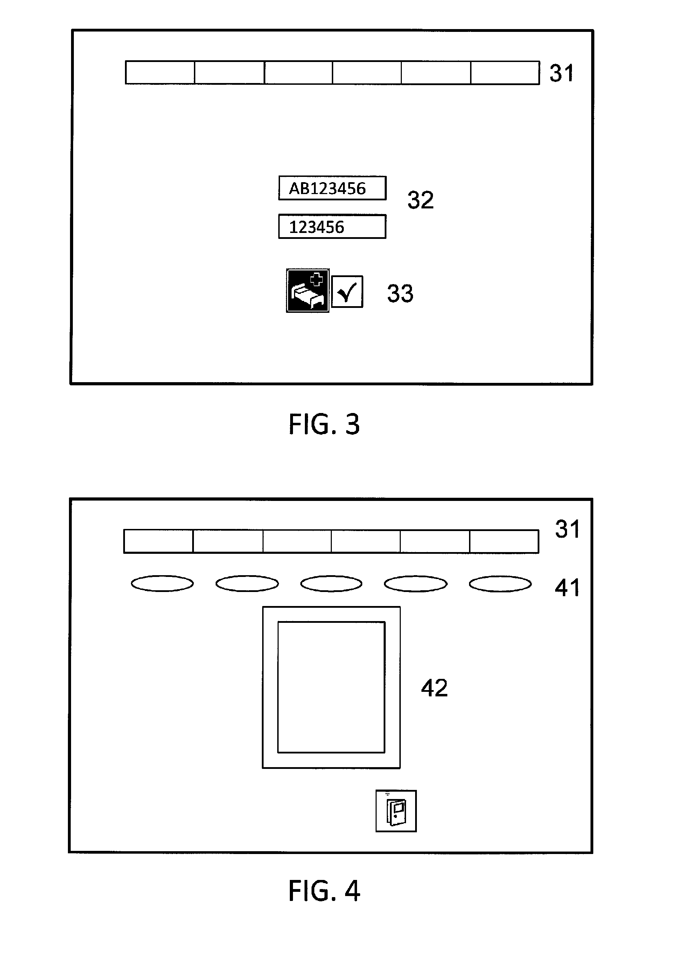

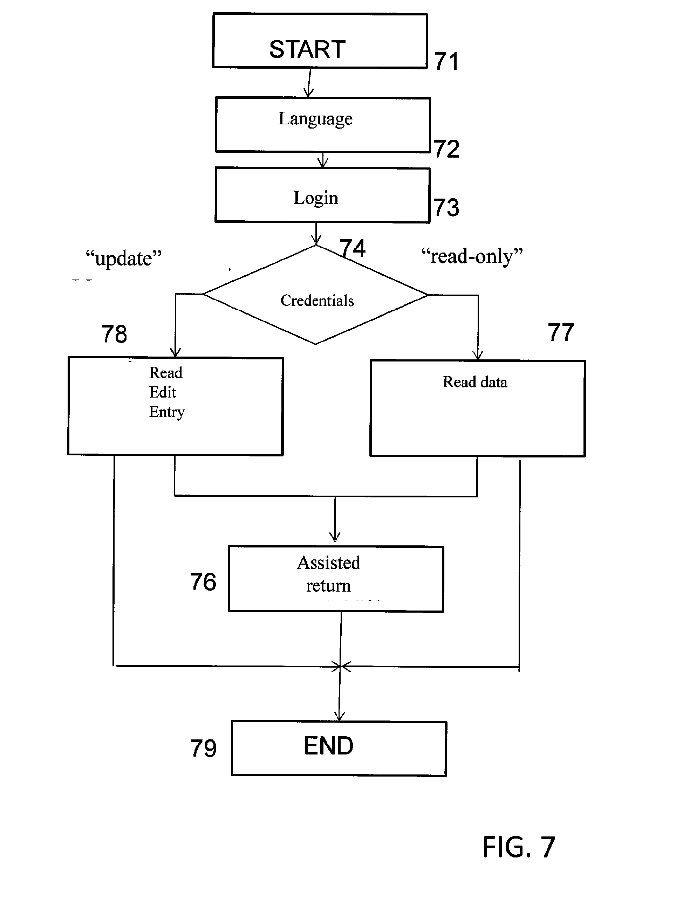

The system is used in accordance with the following sequence of steps (flow chart of Figure 7), which are implemented by means of application programs, some of which reside on the user devices.
- At step 71 (START), access is gained to the service management Internet site, as shown on the card, by either the user or by the sanitary staff addressed by the user, or by assistance personnel, e.g. in sanitary emergency situations, through the electronic unit with which the user or the sanitary staff are equipped.
- At step 72 (Language), once the connection has been established, the language 11, 31 is selected in the initial screen (Figure 1 or 3), which shows some basic information identifying the service.
- At step 73 (Login), the access credentials 12, 32 are entered in the next screen: one may use either the read-only credentials or the full credentials allowing also data entry and editing.
- The read-only credentials can be used after the user has given his/her consent to the processing of his/her data in case of emergency.
- With read-only credentials (test of step 74 - credentials), i.e. first-level credentials as defined above, it is possible to click an icon 33 that will turn on the emergency service manageable by the system, i.e. access to the patient's data with his/her permission, including assisted return.
- In any case, clinical information about the user will appear on the screen (step 77 - read data), as previously defined. Icons 21, 41 that allow choosing the type of information will be shown on the screen, as well as the chosen set of information 22, 42. For emergency service management, this information will be useful to the assistance personnel in order to identify any specific requirements or problems of the user, e.g. necessity or impossibility of adopting certain treatments or administering emergency drugs.
- The activation of the "assisted return" icon starts a request for urgent remote intervention, e.g. by air rescue vehicles (Assisted return, step 76). Assisted return can be activated by phone, or the user's position can be located by the GPS function and a request is then activated.
- If second-level credentials, as defined above, were initially entered (step 74), in addition to the above-defined read-only operations the user can also gain access to the system to edit or enter personal data (step 78), especially clinical data, as previously described, including changing his/her login password (PIN). Furthermore, the user can also activate an assisted return request (step 76).
- After use, the application will be closed (step 79 - END).

The part of the program that resides on the server is an application written in Asp.net, C# and Vb.net, running on IIS, and accessing and editing data recorded in an Sql Server database.

The method and the system of the invention provide automated management of the access to clinical data in emergency conditions, integrated with emergency intervention in any place and at any time. This solves the technical problem of overcoming the manual procedures for associating and collecting information and for making emergency calls, which cannot be carried out quickly.

The invention thus provides an automated system for maintaining clinical records for a plurality of participating patients, which records are remotely accessible for reading and editing each user's clinical record data.

The present invention can advantageously be implemented through computer programs comprising coding means for implementing one or more steps of the above-described method, when such programs are executed by computers. It is therefore understood that the protection scope extends to said computer program as well as to computer-readable means that comprise a recorded message, said computer-readable means comprising program coding means for implementing one or more steps of the method when said program is executed by a computer.

The above-described example of embodiment may be subject to variations without departing from the protection scope of the present invention, including all equivalent designs known to a man skilled in the art.

The elements and features shown in the various preferred embodiments may be combined together without however departing from the protection scope of the present invention. From the above description, those skilled in the art will be able to produce the object of the invention without introducing any further implementation details.

## Claims

1. Method for automated management of patients' clinical data, and for allowing access to said clinical data in the event of sanitary emergencies, through a system of electronic processing units interconnected via the Internet network, said system comprising a server processing unit, the method being **characterized in that** it comprises the steps of:
- providing a user registration system assigning access credentials and a sanitary identification card; said access credentials include a first level of read-only credentials and a second level of read/write credentials;
- providing access to clinical information stored in said server processing unit (51) by means of personal processing units (52, 53, 54), via said Internet network and said first and second levels of credentials;
- storing said clinical information in a multilingual format, allowing said registered users to choose one of said languages for the read/write operations to be executed in said server processing unit (51);
- providing a system for emergency management and assisted return calling through said personal processing units (52, 53, 54) with said first or second level of access credentials.

2. Method for automated management of patients' clinical data and for sanitary emergency management as claimed in claim 1, comprising the step of assigning the following access levels in said server processing unit (51):
- a first level of access through said first level of credentials, for data read-only operations or for emergency calls, by using the identifiers written on said sanitary identification card, said first level being usable by anyone having the possibility of reading said card;
- a second level of access through said second level of credentials, for data read, entry and update operations, or for sanitary return calls, said second level being only available to the registered user, upon modification of said access credentials by said registered user;
- a third level of access for managing said system in said server processing unit.

3. Method for automated management of patients' clinical data and for sanitary emergency management as claimed in claim 1, wherein said storing of clinical information includes entering or updating personal identification data and clinical data, as well as password modification, through said personal processing units (52, 53, 54) with said second level of credentials.

4. Method for automated management of patients' clinical data and for sanitary emergency management as claimed in claim 1, wherein said system for activating sanitary return calls comprises the identification of the position of the calling personal processing unit.

5. System for automated management of patients' clinical data and for sanitary emergency management adapted to implement the method according to any one of claims 1 to 4, comprising said system of electronic processing units interconnected via the Internet network, said sanitary identification cards, and means for executing each one of the steps of the preceding claims.
